# EUROPEAN PATENT SPECIFICATION

(11) **EP 3 600 255 B1**
(45) Date of publication and mention of the grant of the patent: **07.07.2021**
(21) Application number: 18715636.9
(22) Date of filing: 29.03.2018
(51) Int. Cl.: A61K 9/14, A61K 9/20

(54) **STABLE HOT-MELT EXTRUDATE CONTAINING VALSARTAN AND SACUBITRIL**
STABILES HEISSSCHMELZEXTRUDAT MIT VALSARTAN UND SACUBITRIL
EXTRUDAT THERMOFUSIBLE STABLE CONTENANT DU VALSARTAN ET DU SACUBITRIL

(30) Priority: 31.03.2017 IN 201711011677; 01.06.2017 US 201762513605 P
(43) Date of publication of application: 05.02.2020
(73) Proprietor: Alfred E. Tiefenbacher (GmbH & Co. KG), 22767 Hamburg (DE)
(72) Inventor: RALLABANDI, Bala Ramesha Chary, Hyderabad (Telangana) 500090 (IN); PRATHAP, Vamshi Ramana, Jagitial District 505452 (IN); GOLLAPUDI, Rajesh Krishna Mohan, East Godavari District (Andhra Pradesh) 533437 (IN); SCHLEHAHN, Hendrik, 22767 Hamburg (DE)
(74) Representative: Hamm&Wittkopp Patentanwälte PartmbB
(86) International application number: PCT/EP2018/058207
(87) International publication number: WO 2018/178295

(56) References cited:
- WO-A1-2017/037596
- WO-A1-2017/042700
- WO-A1-2018/069937
- LADAN AKBARPOUR NIKGHALB ET AL: "Review Article: Solid Dispersion: Methods and Polymers to increase the solubility of poorly soluble drugs", JOURNAL OF APPLIED PHARMACEUTICAL SCIENCE, vol. 2, no. 10, 1 October 2012 (2012-10-01), pages 170-175, XP55225167, Gwalior DOI: 10.7324/JAPS.2012.21031

## Description

The present invention relates to a solid dispersion containing valsartan and sacubitril as well as to a pharmaceutical composition containing such a solid dispersion.

The combination valsartan and sacubitril is marketed under the tradename Entresto® in the form of film-coated tablets for the prevention of heart failure in patients with chronic heart failure. Entresto® contains the drug combination in the form of a cocrystal consisting of valsartan disodium, sacubitril monosodium and 2.5 molecules water. The cocrystal has been designated as LCZ696; its preparation and physical/chemical properties are described in WO 2007/056546 and in Tetrahedron Letters 2012, 53, 275-276. In the Tetrahedron Letters, it is further reported that a desolvated crystalline form exists because the crystalline structure of LCZ696 is maintained up to the melting temperature (around 138°C), in spite of the fact that two water molecules are lost during the heating.

Various polymorphic forms, pseudopolymorphic forms of LCZ696, i.e. crystalline forms in which the cocrystal contains either more molecules or less molecules of water than 2.5 molecules, and amorphous forms of LCZ696 are known, which are described in WO 2016/037552, WO 2016/049663, WO 2016/051393, WO 2016/125123, WO 2016/151525, WO 2016/201238, WO 2017/009784 and WO 2017/012917.

The Entresto® film-coated tablet is an immediate-release tablet that contains, besides LCZ696, microcrystalline cellulose, low-substituted hydroxypropyl cellulose, crospovidone, magnesium stearate, talc and colloidal silicon dioxide as pharmaceutical excipients. Three strengths of the tablet are marketed, which contain, on the basis of the free acid weight of the drugs, 24 mg/26 mg, 49 mg/51 mg and 97 mg/103 mg of sacubitril/valsartan. WO 2009/061713 discloses an immediate-release tablet containing LCZ696 prepared by direct compression or dry-granulation. In the preparation of the tablet, moisture, excessive heat and high shear forces should be avoided in order to prevent amorphization as well as dissociation of the drug components of LCZ696.

WO 2017/000864 describes a direct compression method for preparing a tablet containing LCZ696, in which a mixture of the drug, a hydrophilic diluent, a binder and a disintegrant is subjected to compression.

WO 2017/012600 discloses a tablet containing a physical mixture of sacubitril or a pharmaceutically acceptable salt thereof and valsartan or a pharmaceutically acceptable salt thereof that can be prepared by direct compression, dry-granulation or wet-granulation. The tablets are very sensitive to moisture, so that packaging under nitrogen atmosphere is recommended in order to prevent the degradation of the drugs.

WO 2017/037596 discloses an amorphous solid dispersion of LCZ696 prepared by rotational distillation, spray-drying or freeze-drying a solution containing LCZ696 and a pharmaceutical excipient such as a polymer or magnesium aluminometasilicate (e.g. Neusilin®).

The objective underlying the present invention was the provision of a solid unit dosage form for oral administration that contains valsartan or a pharmaceutically acceptable salt thereof and sacubitril or a pharmaceutically acceptable salt thereof, optionally in form of a complex of the two active ingredients, in a physically and chemically stable form. It was a further objective of the present invention to provide a physico-mechanical stable solid unit dosage form containing these active ingredients. These objectives are attained by the subject matter as defined in the claims.

The solid unit dosage form of the present invention is an immediate-release solid unit dosage form for oral administration, preferably an optionally film-coated tablet or granules filled in a pouch. The solid unit dosage form contains a solid dispersion comprising valsartan or a pharmaceutically acceptable salt thereof and sacubitril or a pharmaceutically acceptable salt thereof as active ingredients. The active ingredients are dispersed in a matrix containing a polymer, wherein the solid dispersion is prepared by hot-melt extrusion. The expression "solid dispersion" as used herein relates to a drug molecularly dissolved in the solid excipient(s) matrix (solid solution) or a drug dispersed as crystalline or amorphous particles in the solid excipient(s) matrix.

The active ingredients are preferably in a non-crystalline state (i.e. molecularly dissolved or in the form of amorphous particles). The absence of crystalline drug in the solid dispersion can be determined by differential scanning calorimetry (DSC), powder X-ray diffraction (PXRD) and scanning electron microscopy (SEM), respectively. The solid dispersion preferably comprises the active ingredients in a ratio (mol/mol) of 1:1.

According to a preferred embodiment of the present invention, the solid dispersion contains valsartan disodium and sacubitril monosodium, preferably in a ratio (mol/mol) of 1:1. The solid dispersion is prepared by subjecting a mixture containing the polymer and the active ingredients to hot-melt extrusion, wherein the active ingredients are selected from valsartan disodium, sacubitril monosodium and a complex of valsartan disodium and sacubitril monosodium. US 5,217,996 discloses a process for the preparation of sacubitril and its pharmaceutically acceptable salts, in particular, the monosodium salt of sacubitril, and various salts of valsartan, e.g. its disodium salt, are disclosed in WO 02/06253.

For the preparation of the solid dispersion of the present invention, the complex of valsartan disodium and sacubitril monosodium, either in crystalline or amorphous form, can be used in the hot-melt extrusion process. Alternatively, the individual drugs, i.e. valsartan and sacubitril, or pharmaceutically acceptable salts thereof, either in crystalline or amorphous form, may be subjected to the hot-melt extrusion process. Examples of a crystalline complex of valsartan disodium and sacubitril monosodium include LCZ696 or a polymorphic or a pseudopolymorphic form thereof. The expression "pseudopolymorphic form" relates to crystalline hydrates of the complex of valsartan disodium and sacubitril monosodium other than the hemipentahydrate LCZ696, which contain either more water molecules or less water molecules than 2.5 molecules in the crystal lattice.

The solid dispersion of the present invention contains a polymer that is preferably selected from polyvinylpyrrolidone, poly(vinylpyrrolidone/vinylacetate), polyvinylcaprolactam/polyvinylacetate/polyethylene glycol graft copolymer, polyethylene glycol/polyvinyl alcohol graft copolymer, poly(ethylene oxide), poly(ethylene oxide/propylene oxide), macrogolglycerol hydroxystearate, hydroxypropyl methylcellulose, hydroxypropyl cellulose, D-α-tocopheryl polyethylene glycol succinate, poly(butyl methacrylate/2-dimethylaminoethyl methaerylate/methyl methacrylate), poly(ethyl acrylate/methyl methacrylate) and poly(ethyl acrylate/methyl methacrylate/trimethylammonioethyl methacrylate chloride). According to a preferred embodiment of the present invention, the polymer is selected from polyvinylcaprolactam/polyvinylacetate/polyethylene glycol graft copolymer, hydroxypropyl methylcellulose and poly(vinylpyrrolidone/vinylacetate) optionally in admixture with polyethylene glycol. Mixtures of polymers may be contained in the solid dispersion of the present invention.

Sacubitril and their pharmaceutically acceptable salts, such as sacubitril monosodium, as well as the amorphous complex of valsartan disodium and sacubitril monosodium are solid but very hygroscopic substances. These substances become deliquescent and sticky when exposed to air humidity. The polymer constituting the matrix of the solid dispersion of the present invention serves the purpose to protect the active ingredients from moisture. Thus, the present invention also relates to the use of polymers for protecting the active ingredients from moisture-induced chemical and physical (polymorph conversion, recrystallization or amorphization) degradation,

It is well known that the handling and the formulation of hygroscopic and deliquescent, sticky active ingredients into solid pharmaceutical formulations is difficult and requires extensive precautions. When water absorption occurs during manufacturing, the consequences include processing problems such as stickiness, clumping, poor release from punches, poor flow characteristics and poor compressibility. Moreover, the physico-mechanical properties and appearance of solid dosage forms comprising a hygroscopic or deliquescent active ingredient are often insufficient, especially after storage. For example, an insufficient tablet hardness as well as unacceptable crumbling or even liquifying of the solid dosage forms may occur. Thus, the present invention also relates to the use of polymers for reducing or eliminating manufacturing and physico-mechanical stability problems of solid dosage forms, which are associated with the hygroscopicity and deliquescence of sacubitril or salts thereof, such as sacubitril monosodium, or a complex of valsartan disodium and sacubitril monosodium as active ingredients.

The solid dispersion may additionally contain a monomeric plasticizer, e.g. triethylcitrate, triacetin, dibutyl sebacate, diethyl phthalate, glycerylmonostearate, glycerine or propylene glycol, or a polymeric plasticizer such as polyethylene glycol or poly(ethylene oxide/propylene oxide). In order to increase the hygroscopic stability of the solid dispersion, a porous, preferably mesoporous inorganic stabilizer can be incorporated, such as mesoporous silica. A mesoporous material is a material containing pores with diameters between 2 and 50 nm. Suitable mesoporous silica products are commercially available under the tradename Syloid®. As an alternative to mesoporous silica, mesoporous magnesium aluminometasilicate may be used, e.g. the magnesium aluminometasilicates marketed under the tradename Neusilin®. A further alternative is mesoporous magnesium carbonate, which is available under the tradename Upsalite®.

Typically, the solid dispersion contains the active ingredients and the polymer in a weight ratio of 3.5 : 1 to 1 : 3.5, preferably from 2 : 1 to 1 : 1.5 (total weight of the active ingredients, calculated on the basis of the free acid weight of the drugs, to the total weight of the polymer(s)).

The solid dispersion of the present invention is contained in the solid unit dosage form for oral administration together with a pharmaceutical excipient. Preferably, the pharmaceutical excipient is selected from diluents, disintegrants, mesoporous inorganic hygroscopic-stability increasing substances, lubricants and glidants. The solid unit dosage form may be an optionally film-coated tablet. The film coating may be a moisture-barrier film coating in order to increase the hygroscopic stability of the tablet. Alternatively, the milled extrudate may be filled in sachets without additional pharmaceutical excipients.

Examples of diluents include microcrystalline cellulose, calcium hydrogen phosphate, lactose (anhydrous or monohydrate) and calcium carbonate. Examples of disintegrants include croscarmellose sodium, sodium starch glycolate, polyvinylpolypyrrolidone (crospovidone) and low-substituted hydroxypropyl cellulose (L-HPC). As glidants silicon dioxide, talc and the like may be used, while magnesium stearate, calcium stearate, stearic acid, sodium stearyl fumarate and glycerol dibehenate are examples of suitable lubricants. Examples of mesoporous inorganic hygroscopic-stability increasing substances include silica products (e.g. Syloid®), magnesium aluminometasilicate products (e.g., Neusilin®) and magnesium carbonate products (Upsalite®),

The solid unit dosage forms of the present invention are contained in blister packages, bottles or sachets made for example from PVC, PVDC, PCTFE, COC, PET, PA, Alu, PE or PP and combinations or multilayer films thereof. These packages may comprise a moisture barrier layer and/or they may be packed together with desiccants.

The present invention further relates to a process for preparing an optionally film-coated tablet. The process comprises the steps:
i) subjecting
   (a) a mixture containing valsartan or a pharmaceutically acceptable salt thereof, sacubitril or a pharmaceutically acceptable salt thereof, and a polymer to hot-melt extrusion, or
   (b) a mixture containing a complex of valsartan disodium and sacubitril monosodium, and a polymer to hot-melt extrusion, or
   (c) a first mixture containing valsartan or a pharmaceutically acceptable salt thereof and a polymer to hot-melt extrusion to obtain a first extrudate, a second mixture containing sacubitril or a pharmaceutically acceptable salt thereof and a polymer to hot-melt extrusion to obtain a second extrudate, optionally subjecting a mixture containing the first extrudate and the second extrudate to hot-melt extrusion,
ii) milling the extrudate obtained in step (i)(a), (b) or (c) to obtain granules,
iii) preparing a mixture containing the granules obtained in step (ii) and a pharmaceutical excipient,
iv) compressing the mixture obtained in step (iii) into the tablet.

Alternatively, the granules obtained in step (ii) may be filled in sachets. Alternatively, the granules obtained in step (ii) may be filled in capsules, wherein the granules obtained in step (ii) may be optionally mixed with a pharmaceutical excipient before filling in capsules.

Typically, the maximum temperature applied in the hot-melt extrusion is 120 °C to 160 °C, preferably 130 °C to 150 °C and more preferably 135 °C to 145 °C. The hot-melt extrusion has to be carried out at a temperature that allows the dissolution of the active ingredients in the polymer-containing matrix. Optionally, a degassing unit may be employed during hot-melt extrusion processing.

The following examples are intended to further illustrate the present invention.

### Examples

Hot-melt extrusion was performed with a Pharma 11 Twin-screw hot-melt extruder from Thermo Fisher Scientific Inc.

### Examples 1 to 8

| **Ingredients** | **Ex. 1 [mg]** | **Ex. 2 [mg]** | **Ex. 3 [mg]** | **Ex. 4 [mg]** | **Ex.5 [mg]** | **Ex. 6 [mg]** | **Ex. 7 [mg]** | **Ex. 8 [mg]** |
|---|---|---|---|---|---|---|---|---|
| **Stage-A (Dry mix for extrusion)** | | | | | | | | |
| Sacubitril/Valsartan complex (crystalline) | 228.516* | 228,516* | 228.516* | 228.516* | - | - | - | - |
| Sacubitril/Valsartan complex (amorphous) | - | - | - | - | 237.553* | - | 237.553* | 237.553* |
| Sacubitril monosodium | - | - | - | - | - | 103.664* | - | - |
| Valsartan disodium | - | - | - | - | - | 120.649* | - | - |
| HPMC 15 cps | - | - | - | - | - | - | 142.532 | - |
| Copovidone (Kollidon VA 64) | 137.110 | 114.258 | 137.110 | 137.110 | 142.532 | 134.588 | - | 142.532 |
| Polyethylene glycol (PEG 3350) | - | - | - | 13.711 | - | - | - | - |
| Colloidal silica (Syloid® AL-IFP) | - | - | - | - | - | - | 5.000 | 5.000 |

| **Stage-B (Pre-lubrication/ blending)** | | | | | | | | |
|---|---|---|---|---|---|---|---|---|
| Microcrystalline cellulose (Comprecel® PH I02) | 12.374 | 25.226 | 12.374 | 8.663 | 7.915 | 9.099 | 1.915 | 1.915 |
| Low substituted HPC (L-HPC LH11) | 10.000 | 10.000 | 10.000 | 10.000 | 10.000 | 10.000 | 10.000 | 10.000 |
| Crospovidone Type A (Polyplasdone® XL) | 20.000 | 20.000 | 20.000 | 20.000 | 20.000 | 20.000 | 20.000 | 20.000 |
| Colloidal anhydrous silica (Aerosil® 200) | 4.000 | 4.000 | 4.000 | 4.000 | 4.000 | 4.000 | - | - |
| Colloidal silica (Syloid® 244FP) | - | - | - | - | - | - | 9.000 | 9.000 |

| **Stage-B (Lubrication)** | | | | | | | | |
|---|---|---|---|---|---|---|---|---|
| Talc | 4.000 | 4.000 | 4.000 | 4.000 | 4.000 | 4.000 | 4.000 | 4.000 |
| Magnesium stearate | 4.000 | 4.000 | 4.000 | 4.000 | 4.000 | 4.000 | 4.000 | 4.000 |
| **Core Tablet Weight** | **420.000** | **410.000** | **420.000** | **430.000** | **430.000** | **410.000** | **434.000** | **434.000** |
| **Stage-C (Film-Coating)** | | | | | | | | |
| Opadry® 00F540020 Pink | 16.000 | 16.000 | 16.000 | 16.000 | 16.000 | 16.000 | 16.000 | 16.000 |
| Water, Purified | q.s. | q.s. | q.s. | q.s. | q.s. | q.s. | q.s. | q.s. |
| **Film-Coated Tablet Weight** | **436.000** | **426.000** | **436.000** | **446.000** | **416.000** | **426.000** | **450.000** | **450.000** |

| **Zone [°C]** | | | | | | | | |
|---|---|---|---|---|---|---|---|---|
| Z2 | 40 | | 40 | | | | | |
| Z3 | 70 | | 70 | | | | | |
| Z4 | 100 | | 100 | | | | | |
| Z5 | 120 | | 120 | | | | | |
| Z6 | 150 | | 140 | | | | | |
| Z7 | 140 | | 130 | | | | | |
| Z8 | 130 | | 120 | | | | | |
| **Die [°C]** | 120 | | 120 | | | | | |
| **Feed rate** | Manual | | | | | | | |
| **Screw [rpm]** | 150 | 150 | 150 | 150 | 150 | 150 | 150 | 150 |
| **Die pressure [?]** | 6 | 7 | 4 | 3 | 21 | 16 | 12 | 16 |
| **Melt temp. [°C]** | 125 | 124 | 124 | 123 | 122 | 122 | 121 | 123 |
| **Torque [%]** | 36 | 39 | 35 | 29 | 43 | 51 | 52 | 75 |

| | | | | | | | | |
|---|---|---|---|---|---|---|---|---|
| *Contain 97 mg sacubitril and 103 mg valsartan, respectively | | | | | | | | |

### Process:

1. Stage A materials were sifted through #35 mesh, added into double cone blender and blended for 5 minutes and subsequently processed in HME.
2. The extrudes were milled through 1575 µ, 1143 µ and finally 610 µ-screens.
3. Crospovidone, L-HPC, microcrystalline cellulose and colloidal anhydrous silica were sifted through #35 mesh and blended with milled extrudes in double cone blender for 10 minutes.
4. Magnesium stearate and talc were sifted through 35# mesh and blended with step 3 material in double cone blender for 5 minutes.
5. Step 4 lubricated blend was compressed into tablets on rotary tablet compression machine using suitable tooling and parameters.
6. Core tablets were coated in coating pan using Opadry® 00F540020 Pink suspension using Water as solvent.

The extrudes showed a good grindability. The milled extrudes contained the active ingredients in non-crystalline state. The physical and chemical stability was high. The milled extrudes showed good compressibility. No crystalline drugs could be detected in the tablets after storage for two months at 40 °C/75% RH in a clear PVC-PVDC packaging, and the chemical stability of the drugs was high. The physico-mechanical stability of the tablets before and after storage for two months at 40 °C/75% RH in a clear PVC-PVDC packaging was high.

## Claims

1. A solid dispersion comprising valsartan or a pharmaceutically acceptable salt thereof and sacubitril or a pharmaceutically acceptable salt thereof as active ingredients, wherein the active ingredients are dispersed in a matrix containing a polymer, and wherein the solid dispersion is prepared by hot-melt extrusion.

2. The solid dispersion according to claim 1, wherein the active ingredients are in a non-crystalline state.

3. The solid dispersion according to claim 1 or 2, wherein the solid dispersion comprises the active ingredients in a ratio (mol/mol) of 1 : 1.

4. The solid dispersion according to according to any one of the preceding claims, wherein the active ingredients are valsartan disodium and sacubitril monosodium.

5. The solid dispersion according to claim 4, wherein the solid dispersion is prepared by subjecting a mixture containing the polymer and the active ingredients to hot-melt extrusion, wherein the active ingredients are selected from valsartan disodium, sacubitril monosodium and a complex of valsartan disodium and sacubitril monosodium.

6. The solid dispersion according to claim 5, wherein the complex of valsartan disodium and sacubitril monosodium is crystalline or amorphous.

7. The solid dispersion according to claim 6, wherein the crystalline complex of valsartan disodium and sacubitril monosodium is LCZ696 or a polymorphic or a pseudopolymorphic form thereof.

8. The solid dispersion according to any one of the preceding claims, wherein the polymer is selected from polyvinylpyrrolidone, poly(vinylpyrrolidone/vinyl acetate), polyvinylcaprolactam/polyvinylacetate/polyethylene glycol graft copolymer, polyethylene glycol/polyvinyl alcohol graft copolymer, poly(ethylene oxide), poly(ethylene oxide/propylene oxide), macrogolglycerol hydroxystearate, hydroxypropyl methyl cellulose, hydroxypropyl cellulose, D-α-tocopheryl polyethylene glycol succinate, poly(butyl methacrylate/2-dimethylaminoethyl methacrylate/methyl methacrylate), poly(ethyl acrylate/methyl methacrylate) and poly(ethyl acrylate/methyl methacrylate/trimethylammonioethyl methacrylate chloride).

9. The solid dispersion according to claim 8, wherein the polymer is polyvinylcaprolactam/polyvinylacetate/polyethylene glycol graft copolymer, hydroxypropyl methylcellulose or poly(vinylpyrrolidone/vinylacetate) optionally in admixture with polyethylene glycol, preferably poly(vinylpyrrolidone/vinylacetate) or hydroxypropyl methylcellulose.

10. A solid unit dosage form for oral administration comprising the solid dispersion according to any one of the preceding claims and a pharmaceutical excipient.

11. The solid unit dosage form according to claim 10, wherein the pharmaceutical excipient is selected from diluents, disintegrants, lubricants, glidants and mesoporous inorganic hygroscopic-stability increasing substances.

12. The solid unit dosage form according to claim 10 or 11, wherein the solid unit dosage form is an optionally film-coated tablet.

13. A process for preparing an optionally film-coated tablet according to claim 11, comprising the steps:
i) subjecting
(a) a mixture containing valsartan or a pharmaceutically acceptable salt thereof, sacubitril or a pharmaceutically acceptable salt thereof, and a polymer to hot-melt extrusion, or
(b) a mixture containing a complex of valsartan disodium and sacubitril monosodium, and a polymer to hot-melt extrusion, or
(c) a first mixture containing valsartan or a pharmaceutically acceptable salt thereof and a polymer to hot-melt extrusion to obtain a first extrudate, a second mixture containing sacubitril or a pharmaceutically acceptable salt thereof and a polymer to hot-melt extrusion to obtain a second extrudate, optionally subjecting a mixture containing the first extrudate and the second extrudate to hot-melt extrusion,
ii) milling the extrudate obtained in step (i)(a), (b) or (c) to obtain granules,
iii) preparing a mixture containing the granules obtained in step (ii) and a pharmaceutical excipient,
iv) compressing the mixture obtained in step (iii) into the tablet.

## Patentansprüche

1. Feste Dispersion umfassend Valsartan oder ein pharmazeutisch verträgliches Salz davon und Sacubitril oder ein pharmazeutisch verträgliches Salz davon als Wirkstoffe, wobei die Wirkstoffe in einer Matrix dispergiert sind, die ein Polymer enthält, und wobei die feste Dispersion durch Heißschmelzextrusion hergestellt ist.

2. Feste Dispersion nach Anspruch 1, wobei sich die Wirkstoffe in einem nichtkristallinen Zustand befinden.

3. Feste Dispersion nach Anspruch 1 oder 2, wobei die feste Dispersion die Wirkstoffe in einem Verhältnis (Mol/Mol) von 1 : 1 umfasst.

4. Feste Dispersion nach einem der vorhergehenden Ansprüche, wobei die Wirkstoffe Valsartan-Dinatrium und Sacubitril-Mononatrium sind.

5. Feste Dispersion nach Anspruch 4, wobei die feste Dispersion hergestellt wird, indem eine Mischung, die das Polymer und die Wirkstoffe enthält, einer Heißschmelzextrusion unterzogen wird, wobei die Wirkstoffe ausgewählt sind aus Valsartan-Dinatrium, Sacubitril-Mononatrium und einem Komplex von Valsartan-Dinatrium und Sacubitril-Mononatrium.

6. Feste Dispersion nach Anspruch 5, wobei der Komplex aus Valsartan Dinatrium und Sacubitril-Mononatrium kristallin oder amorph ist.

7. Feste Dispersion nach Anspruch 6, wobei der kristalline Komplex von Valsartan-Dinatrium und Sacubitril-Mononatrium LCZ696 oder eine polymorphe oder eine pseudopolymorphe Form davon ist.

8. Feste Dispersion nach einem der vorhergehenden Ansprüche, wobei das Polymer ausgewählt ist aus Polyvinylpyrrolidon, Poly(Vinylpyrrolidon/ Vinylacetat), Polyvinylcaprolactam/Polyvinylacetat/Polyethylenglykol-Pfropfcopolymer, Polyethylenglykol/Polyvinylalkohol-Pfropfcopolymer, Polyethylenoxid, Poly(Ethylenoxid/Propylenoxid), Makrogolglycerinhydroxystearat, Hydroxypropylmethylcellulose, Hydroxypropylcellulose, D-α-Tocopherylpolyethylenglykolsuccinat, Poly(Butylmethacrylat/2-Dimethylaminoethylmethacrylat/Methylmethacrylat), Poly(Ethylacrylat/Methylmethacrylat) und Poly(Ethylacrylat/Methylmethacrylat/ Trimethylammonioethylmethacrylatchlorid).

9. Feste Dispersion nach Anspruch 8, wobei das Polymer Polyvinylcaprolactam/Polyvinylacetat/Polyethylenglykol-Pfropfcopolymer, Hydroxypropylmethylcellulose oder Poly(Vinylpyrrolidon/Vinylacetat) ist, gegebenenfalls im Gemisch mit Polyethylenglykol, vorzugsweise Poly(Vinylpyrrolidon/Vinylacetat) oder Hydroxypropylmethylcellulose.

10. Feste Darreichungsform zur oralen Verabreichung umfassend die feste Dispersion gemäß einem der vorhergehenden Ansprüche und einen pharmazeutischen Hilfsstoff.

11. Feste Darreichungsform nach Anspruch 10, wobei der pharmazeutische Hilfsstoff ausgewählt ist aus Verdünnungsmitteln, Sprengmitteln, Schmiermitteln, Gleitmitteln und mesoporösen anorganischen Substanzen, die die hygroskopische Stabilität erhöhen.

12. Feste Darreichungsform nach Anspruch 10 oder 11, wobei die feste Darreichungsform eine gegebenenfalls filmbeschichtete Tablette ist.

13. Verfahren zur Herstellung einer gegebenenfalls filmbeschichteten Tablette nach Anspruch 11, umfassend die Schritte:
i) Durchführung einer Heißschmelzextrusion
(a) mit einer Mischung, die Valsartan oder ein pharmazeutisch verträgliches Salz davon, Sacubitril oder ein pharmazeutisch verträgliches Salz davon und ein Polymer enthält, oder
(b) mit einer Mischung, die einen Komplex aus Valsartan-Dinatrium und Sacubitril-Mononatrium und ein Polymer enthält, oder
(c) mit einer ersten Mischung, die Valsartan oder ein pharmazeutisch verträgliches Salz davon und ein Polymer enthält, um ein erstes Extrudat zu erhalten, mit einer zweiten Mischung, die Sacubitril oder ein pharmazeutisch verträgliches Salz davon und ein Polymer enthält, um ein zweites Extrudat zu erhalten, gegebenenfalls mit einer Mischung, die das erste Extrudat und das zweite Extrudat enthält;
ii) Mahlen des in Schritt (i)(a), (b) oder (c) erhaltenen Extrudats, um ein Granulat zu erhalten,
iii)Herstellen einer Mischung, die das in Schritt (ii) erhaltene Granulat und einen pharmazeutischen Hilfsstoff enthält,
iv)Komprimieren der in Schritt (iii) erhaltenen Mischung zu der Tablette.

## Revendications

1. Une dispersion solide comprenant du valsartan ou un sel pharmaceutiqement acceptable de celui-ci et du sacubitril ou un sel pharmaceutiquement acceptable de celui-ci en tant que principes actifs, dans laquelle les principes actifs sont dispersés dans une matrice contenant un polymère, et dans laquelle la dispersion solide est préparée par extrusion avec thermo-fusion.

2. La dispersion solide selon la revendication 1, dans laquelle les principes actifs sont à l'état non cristallin.

3. La dispersion solide selon la revendication 1 ou 2, dans laquelle la dispersion solide comprend les principes actifs dans une proportion (mol / mol) de 1 : 1.

4. La dispersion solide selon l'une quelconque des revendications précédentes, dans laquelle les principes actifs sont le valsartan disodique et le sacubitril monosodique.

5. La dispersion solide selon la revendication 4, dans laquelle la dispersion solide est préparée en soumettant un mélange contenant le polymère et les principes actifs à une extrusion avec thermo-fusion, dans laquelle les principes actifs sont choisis parmi le valsartan disodique, le sacubitril monosodique et un complexe de valsartan disodique et sacubitril monosodique.

6. La dispersion solide selon la revendication 5, dans laquelle le complexe de valsartan disodique et de sacubitril monosodique est cristallin ou amorphe.

7. La dispersion solide selon la revendication 6, dans laquelle le complexe cristallin de valsartan disodique et de sacubitril monosodique est LCZ696 ou une forme polymorphe ou une forme pseudo-polymorphe de celui-ci.

8. La dispersion solide selon l'une quelconque des revendications précédentes, dans laquelle le polymère est choisi parmi la polyvinylpyrrolidone, le poly(vinylpyrrolidone/vinylacétate), le copolymère greffé polyvinylcaprolactame/ polyvinylacétate/polyéthylèneglycol, le copolymère greffé polyéthylèneglycol/alcool polyvinylique, le poly(éthylène oxyde), le poly(éthylène oxyde/propylène oxyde), l'hydroxystéarate de macrogolglycérol, l'hydroxypropylméthylcellulose, l'hydroxypropylcellulose, le succinate de D-α-tocophérylpolyéthylèneglycol, le poly(méthacrylate de butyle/méthacrylate de 2-diméthylaminoéthyle/méthacrylate de méthyle), le poly(acrylate d'éthyle/méthacrylate de méthyle) et poly(acrylate d'éthyle/ méthacrylate de méthyle/chlorure de méthacrylate de triméthylammonioéthyle).

9. La dispersion solide selon la revendication 8, **caractérisée en ce que** le polymère est un copolymère greffé polyvinylcaprolactame/polyvinylacétate/polyéthylèneglycol, un hydroxypropylméthylcellulose ou un poly(vinylpyrrolidone/ vinylacétate) éventuellement en mélange avec du polyéthylèneglycol, de préférence du poly(vinylpyrrolidone/vinylacétate) ou de l'hydroxypropylméthylcellulose.

10. Une forme posologique unitaire solide pour l'administration orale comprenant la dispersion solide selon l'une quelconque des revendications précédentes et un excipient pharmaceutique.

11. La forme posologique unitaire solide selon la revendication 10, dans laquelle l'excipient pharmaceutique est choisi parmi les diluants, les désintégrants, les lubrifiants, les glissants et les substances mésoporeuses inorganiques ce qui augmentent la stabilité hygroscopique.

12. La forme posologique unitaire solide selon la revendication 10 ou 11, dans laquelle la forme posologique unitaire solide est un comprimé éventuellement pelliculé.

13. Procédé de préparation d'un comprimé éventuellement pelliculé selon la revendication 11, comprenant les étapes:
i) subir
(a) un mélange contenant du valsartan ou un sel pharmaceutiquement acceptable de celui-ci, du sacubitril ou un sel pharmaceutiquement acceptable de celui-ci, et un polymère à l'extrusion avec thermo-fusion, ou
(b) un mélange contenant un complexe de valsartan disodique et de sacubitril monosodique, et un polymère à l'extrusion avec thermo-fusion, ou
(c) un premier mélange contenant du valsartan ou un sel pharmaceutiquement acceptable de celui-ci et un polymère à extrusion avec thermo-fusion pour obtenir un premier extrudat, un second mélange contenant du sacubitril ou un sel pharmaceutiquement acceptable de celui-ci et un polymère à extrusion avec thermo-fusion pour obtenir un deuxième extrudat, soumettant éventuellement un mélange contenant le premier extrudat et le deuxième extrudat à l'extrusion avec thermo-fusion,
ii) broyage de l'extrudat obtenu à l'étape (i)(a), (b) ou (c) pour obtenir des granulés,
iii) préparation un mélange contenant les granulés obtenus à l'étape (ii) et un excipient pharmaceutique,
iv) compression du mélange obtenu à l'étape (iii) dans le comprimé.
